(19) Europäisches Patentamt
European Patent Office
Office européen des brevets

(11) Veröffentlichungsnummer : **0 022 532**
**B1**

(12) # EUROPÄISCHE PATENTSCHRIFT

(45) Veröffentlichungstag der Patentschrift : **16.02.83**

(51) Int. Cl.³ : **C 07 C 85/06**, C 07 C 87/123

(21) Anmeldenummer : **80103848.0**

(22) Anmeldetag : **07.07.80**

(54) **Verfahren zur Herstellung von Neopentylamin.**

(30) Priorität : **17.07.79 DE 2928742**

(43) Veröffentlichungstag der Anmeldung : **21.01.81 Patentblatt 81/03**

(45) Bekanntmachung des Hinweises auf die Patenterteilung : **16.02.83 Patentblatt 83/07**

(84) Benannte Vertragsstaaten : **BE CH DE FR GB IT LI**

(56) Entgegenhaltungen :
**DE A 1 493 781**
**FR A 2 292 697**
**GB A 1 074 603**
**EUGEN MÜLLER : « Methoden der Organischen Chemie (Houben Weyl) », (4.) Band XI/I : Stickstoffverbindungen II, Georg Thieme Verlag, S. 126-134 Stuttgart, DE**

(73) Patentinhaber : **BAYER AG**
**Zentralbereich Patente, Marken und Lizenzen**
**D-5090 Leverkusen 1, Bayerwerk (DE)**

(72) Erfinder : **Werner, Friedrich, Dr.**
**Petersenstrasse 1**
**D-5000 Köln 91 (DE)**
Erfinder : **Blank, Heinz Ulrich, Dr.**
**Am Geusfeld 35**
**D-5068 Odenthal (DE)**
Erfinder : **Cramm, Günther, Dr.**
**Höhenstrasse 146**
**D-5090 Leverkusen 3 (DE)**
Erfinder : **Braden, Rudolf, Dr.**
**Nothauser Feld 1**
**D-5068 Odenthal (DE)**
Erfinder : **Ziemann, Heinz, Dr.**
**Am Wiesenberg 10**
**D-5653 Leichlingen 1 (DE)**

**EP 0 022 532 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Verfahren zur Herstellung von Neopentylamin

Die Erfindung betrifft ein Verfahren zur Herstellung von Neopentylamin.

Neopentylamin ist bekannt und beispielsweise durch Hydrierung von Trimethylacetaldoxim an Raney-Nickel (J. Am. Chem. Soc. *60,* 657 (1938)), durch Reduktion von Pivalonitril mit Lithiumalanat in Ether (J. Am. Chem. Soc. *74,* 4052 (1952)), durch den Hofmann'schen Abbau von tert.-Butylacetamid (J. Am. Chem. Soc. *71,* 2808 (1949)), bzw. durch Reduktion von Trimethylacetamid mit Lithiumaluminiumhydrid (J. Am. Chem. Soc. *81,* 3728 (1959)) oder mit Diboran (J. Am. Chem. Soc. *86,* 3566 (1964)) erhalten worden. Für eine technische Durchführung sind diese Synthesen unzweckmäßig, da sie Ausgangsstoffe benutzen, die selbst nur schwer und in schlechten Ausbeuten zugänglich sind.

Weiterhin ist es bekannt, aliphatische Alkohole, wie beispielsweise Ethanol oder Butanol, mit einem etwa 6-fachen molaren Überschuß an Ammoniak, in Gegenwart von etwa 6 bis 9 Mol Wasserstoff pro Mol Alkohol an pelletisiertem Nickel in der Gasphase zu einem Gemischder entsprechenden Mono-, Di- und Tri-Alkylamine umzusetzen, wobei für die 3 verschieden hoch alkylierten Amine Ausbeuten von 25 %, 45 % und 10 bis 15 % der theoretischen Ausbeute erzielt werden (US 2 365 721). Ein weiteres Verfahren zur Aminierung von aliphatischen Alkoholen arbeitet in der Gasphase an einem Skelett-Kupfer-Katalysator, der vor der Reaktion mit Bariumhydroxid vorbehandelt wird, und erzielt beispielsweise bei der Reaktion von Ethanol mit 0,9 Mol Ammoniak und 4,5 Mol Wasserstoff, jeweils pro 1 Mol Ethanol ein Reaktionsgemisch, das 13 % Monoethylamin, 35 % Diethylamin und 19 % Triethylamin enthält.

Aus DE-OS 14 93 781 ist es bekannt, sekundäre Alkohole mit Ammoniak in Gegenwart geringer Mengen Wasserstoff an einem Nickel-, Kobalt- oder Kupferchromitkatalysator zu dem zugehörigen Amin umzusetzen. Es wird beschrieben, daß diese Umsetzung nicht in wirksamer Weise mit einem primären Alkohol durchgeführt werden kann, weiterhin, daß primäre Alkohole unter scharfen Reaktionsbedingungen in unerwünschte Kondensations- und Zersetzungsprodukte umgewandelt werden.

Auch GB 1 074 603 beschreibt die Umsetzung von sekundären Alkoholen mit Ammoniak in Gegenwart von Wasserstoff an Nickel-, Kobalt- oder Kupferkatalysatoren in flüssiger Phase zu den zugehörigen Aminen ; hierin wird weiter darauf verwiesen, daß bei der Gasphasenreaktion von primären Alkoholen mit Ammoniak ein Gemisch der primären, sekundären und tertiären Amine erhalten wird.

In DE-OS 24 56 006 wird die Herstellung von isomerenfreiem 3-Methylbutylamin in einer Aminierungsreaktion mit Ammoniak, in Gegenwart von Wasserstoff an Hydrierkatalysatoren beschrieben, das dadurch ausgezeichnet ist, daß man ein durch thermische Anlagerung von Formaldehyd an Isobuten und anschließende Hydrierung gewonnenes 3-Methylbutanol einsetzt. Dieses Verfahren ist auf die Isomerenfreiheit dieses speziellen primären Amins gerichtet, es enthält keine Angaben über die mitentstehenden sekundären und tertiären Amine. Um Ausbeuten an primärem Amin zu erzielen, wird ein 15 bis 20 molarer Ammoniak-Überschuß, bezogen auf die Alkoholmenge, beschrieben. Im Ausführungsbeispiel ist zur Erzielung von 85 % Ausbeute an 3-Methylbutylamin sogar ein Verhältnis von $NH_3$ : Alkohol wie 22 : 1 erforderlich. Über die Menge an höher alkylierten Aminen fehlt eine Aussage.

In Houben-Weyl, Methoden der Organischen Chemie, 4. Aufl., Band XI/1, Seite 127, letzter Absatz bis S. 128, 1. Absatz wird auf die Dimerisierung des Alkohols unter Wasserabspaltung als Nebenreaktion hingewiesen. Diese Reaktion ist besonders bei primären Alkoholen ausgeprägt. Auf S. 128, 2. Absatz dieser Literaturstelle wird auf die Notwendigkeit der Gegenwart von Wasserstoff bei der Aminierung sekundärer cyclischer und leicht aromatisierbarer Alkohole, beispielsweise des 1, 2, 3, 4-Tetrahydro-2-naphthols, hingewiesen, um Nebenproduktbildung durch Aromatisierung zu vermeiden.

Es wurde nun ein Verfahren zur Herstellung von Neopentylamin, das praktisch frei von höher alkylierten Aminen ist, durch Aminierung mit Ammoniak an Hydrier-/Dehydrierkatalysatoren bei erhöhter Temperatur, gegebenenfalls erhöhtem Druck und gegebenenfalls in Gegenwart von Wasserstoff gefunden, das dadurch gekennzeichnet ist, daß man Neopentanol mit 0,5-10 Mol Ammoniak umsetzt.

Neopentanol ist bekannt und kann beispielsweise durch Reaktion von Wasserstoffperoxid mit Diisobutylen in Gegenwart von Schwefelsäure hergestellt werden (J. Am. Chem. Soc. 77, 3139 (1955)).

Ammoniak kann im erfindungsgemäßen Verfahren in Form einer Lösung, beispielsweise einer wäßrigen Lösung, oder als freies Ammoniak, wie gasförmiges oder flüssiges Ammoniak, eingesetzt werden. Bevorzugt wird Ammoniak als verflüssigtes Ammoniak eingesetzt. Ammoniak kann im erfindungsgemäßen Verfahren beispielweise in einer Menge von 0,5 bis 10 Mol, bevorzugt 1 bis 5 Mol pro Mol Neopentanol eingesetzt werden. Das bei der Reaktion nicht umgesetzte Ammoniak kann aus dem Reaktionsgemisch wiedergewonnen werden und in die erfindungsgemäße Reaktion zurückgeführt werden. Es ist selbstverständlich möglich, noch größere als die angegebenen Mengen Ammoniak einzusetzen, jedoch ist dies aus wirtschaftlichen Gründen nicht vorteilhaft.

Das erfindungsgemäße Verfahren wird beispielsweise bei einer Temperatur von 200 bis 300 °C, bevorzugt von 220 bis 280 °C durchgeführt. Bei der Wahl der Reaktionstemperatur innerhalb des angegebenen Bereiches können die übrigen Verfahrensparameter, wie Aktivität des Hydrierungskatalysators, gewünschter Umsatz oder vorgegebene Verweilzeit, Berücksichtigung finden. So kann beispielsweise bei einem kleineren angestrebten Umsatz, einem stärker aktiven Kontakt und/oder einer längeren vorgegebenen Verweilzeit bei einer tieferen Temperatur innerhalb des angegebenen Bereiches gearbeitet werden. Umgekehrt ermöglicht eine höhere Temperatur entweder einen höheren Umsatz und/oder eine

kürzere Verweilzeit und/oder die Verwendung eines weniger aktiven Hydrierungskatalysators. Beispielsweise wurde für einen ca. 50 %igen Umsatz an gepilltem Raney-Nickel bei einer Verweilzeit von 10 Minuten eine Reaktionstemperatur von 230 °C als geeignet gefunden.

Die Einhaltung eines bestimmten Druckes ist für den Erfolg des erfindungsgemäßen Verfahrens unkritisch, so daß bei vermindertem Druck, bei normalem Druck oder bei erhöhtem Druck gearbeitet werden kann. Die bevorzugte Arbeitsweise für das erfindungsgemäße Verfahren ist die bei erhöhtem Druck. Als erhöhter Druck sei beispielsweise ein Druck von 10 bis 500 bar, bevorzugt 20 bis 300 bar, genannt.

Das erfindungsgemäße Verfahren kann sowohl in der Gasphase als auch in der Flüssigphase durchgeführt werden. Die bevorzugte Durchführung ist die in der Flüssigphase. Besonders bevorzugt ist die Durchführung in der Flüssigphase bei erhöhtem Druck.

Das erfindungsgemäße Verfahren kann sowohl diskontinuierlich als auch kontinuierlich durchgeführt werden. Die bevorzugte Ausführungsart ist die kontinuierliche.

Das erfindungsgemäße Verfahren wird in Gegenwart eines Katalysators durchgeführt, der für die Katalyse von Hydrierungen, bzw. Dehydrierungen eingesetzt werden kann und im weiteren Hydrierungskatalysator genannt wird. Als Hydrierungskatalysator sei beispielsweise ein solcher genannt, der mindestens ein Metall der VIII. Gruppe des Periodensystems (Mendelejew) und/oder Kupfer oder mindestens eines dieser Metalle in Kombination mit mindestens einem Metall aus der Gruppe Vanadin, Chrom und Mangan in metallischer und/oder oxidischer Form enthält. Der Hydrierungskatalysator kann zusammen mit einem inerten Trägermaterial oder ohne Trägermaterial zur Anwendung gelangen. Beispiele für inerte Trägermaterialien sind synthetische und natürlich vorkommende, gegebenenfalls physikalisch oder chemisch veränderte Stoffe wie Aluminiumoxide, Kieselsäure, Kieselgur, Silikate, Aluminiumsilikate, Montmorillionit, Zeolithe, Spinelle, Kaolin, Ton, Magnesiumsilikat, Asbest, Bimstein, Dolomit, Erdalkalicarbonate, Erdalkalisulfate, Zinkoxid, Zirkonoxid, Soliciumcarbid, Borphosphat, Aluminiumphosphat oder Aktivkohle. Solche Trägerkatalysatoren enthalten im allgemeinen etwa 1 bis 70, vorzugsweise 5 bis 65 Gew.-% des katalytisch aktiven Metalls, bezogen auf die Gesamtmasse des Trägerkatalysators. Die katalytisch aktiven Metalle können homogen im Trägermaterial verteilt oder in der äußeren Schicht oder auf der Oberfläche des Trägers gelagert sein.

Die Katalysatoren können ferner einen oder mehrere Beschleuniger oder Aktivatoren, wie Lithium, Natrium, Kalium, Calcium, Barium, Silber, Gold, Beryllium, Lanthan, Cer, Vanadin, Niob, Tantal, Molybdän oder Wolfram in Mengen bis zu 10 Gew.-%, bevorzugt in Mengen bis zu 1 Gew.-% enthalten.

Als aktive Stoffe in metallischer oder oxidischer Form für die erfindungsgemäß einsetzbaren Hydrierungskatalysatoren seien beispielsweise genannt : Palladium, Platin, Ruthenium, Rhodium, Nickel, Kobalt, Eisen oder Kupfer. Die genannten Metalle können sowohl einzeln, als auch als Gemisch mehrerer im erfindungsgemäßen Hydrierungskatalysator enthalten sein. Sie können ferner mit einem Metall aus der Gruppe Aluminium, Vanadin, Chrom und Mangan kombiniert sein. Bevorzugte Hydrierungskatalysatoren für das erfindungsgemäße Verfahren sind solche, die Nickel in metallischer oder oxidischer Form allein oder in Kombination mit mindestens einem der genannten Metalle enthalten, beispielsweise Katalysatoren vom Raney-Typ, wie Raney-Nickel, Raney-Nickel-Eisen, Raney-Nickel-Kobalt, Raney-Nickel-Kupfer, durch Reduktion von Nickelsalzen mit Zinstaub, Alkalihydrid, Borananten, Borwasserstoff, Metallalkylverbindungen oder Hydrazin hergestelltes metallisches Nickel, wie Urushibara-Nickel, durch Reduktion von Nickeloxid oder Gemischen aus Nickeloxid und mindestens einem weiteren Metalloxid mit Wasserstoff hergestellte metallische Katalysatoren, Nickeloxid oder Gemische aus Nickeloxid mit mindestens einem weiteren Metalloxid, wie Nickeloxid-Chromoxid, Nickeloxid-Manganoxid-Kupferoxid, Nickeloxid-Chromoxid-Kupferoxid, oder Trägerkatalysatoren, wie Nickel auf Kieselgur, Nickel auf Aluminiumoxid, Nickel-Kupfer auf Aluminiumoxid oder Nickel-Mangan auf Aluminiumoxid.

Besonders bevorzugte Katalysatoren sind Raney-Nickel Raney-Nickel-Eisen, Raney-Nickel-Kobalt, Raney-Nickel-Kupfer Nickel auf Aluminiumoxid, Nickel auf Kieselgur Nickel, sowie Nickeloxid-Chromoxid.

Die genannten Katalysatoren können einzeln oder im Gemisch aus 2 oder mehreren der genannten Katalysatoren verwendet werden. Die Menge, in der der Katalysator oder das Katalysatorgemisch verwendet wird, kann in weiten Grenzen schwanken. Beispielsweise sei eine Menge von 1 bis 100 Gew.-%, bevorzugt von 5 bis 50 Gew.-% Katalysatormetall, bezogen auf die Menge des eingesetzten Neopentanols, bei einer diskontinuierlichen Arbeitsweise genannt. Für die kontinuierliche Arbeitsweise sei beispielsweise eine Katalysatormenge von 5 bis 500 Gew.-%, bevorzugt 50 bis 250 Gew.-% Katalysatormetall, bezogen auf pro Stunde eingesetztes Neopentanol, genannt. Die genannten Hydrierungskatalysatoren können im erfindungsgemäßen Verfahren im diskontinuierlichen Betrieb wiederholt eingesetzt werden. Bei der Durchführung in kontinuierlicher Arbeitsweise zeigen sie hervorrangend lange Standzeiten. Dadurch ist auch die Verwendung hoher Katalysatorkonzentrationen innerhalb der angegebenen Bereiche durchaus wirtschaftlich.

Das erfindungsgemäße Verfahren ist grundsätzlich ohne oder mit gleichzeitiger Verwendung von Wasserstoff durchführbar. Die Durchführung des Verfahrens in Gegenwart von Wasserstoff ist die bevorzugte Variante, da hierbei besonders hohe Katalysatorstandzeiten und eine besonders hohe Selektivität in Bezug auf die Umsetzung von Neopentanol zu Neopentylamin erzielt wird. Die Verwendung eines großen Überschusses an Wasserstoff, bezogen auf das eingesetzte Neopentanol, ist für das

erfindungsgemäße Verfahren unschädlich. Jedoch ist ein großer Überschuß an Wasserstoff für ein technisches Verfahren nicht zweckmäßig. Es ist daher besonders günstig, daß das erfindungsgemäße Verfahren bereits in Gegenwart katalytischer Mengen Wasserstoff die geschilderten Vorteile der hohen Katalysator-Standzeit und der hohen Selektivität zeigt. Als katalytische Menge Wasserstoff sei beispielsweise 0,001 bis 1 Mol, bevorzugt 0,001 bis 0,5 Mol, bezogen auf eingesetztes Neopentanol, genannt. Besonders bevorzugt ist eine Wasserstoffmenge zwischen 0,01 und 0,1 Mol pro Mol Neopentanol.

Der zur Durchführung des erfindungsgemäßen Verfahrens gegebenenfalls eingesetzte Wasserstoff kann als molekularer Wasserstoff eingesetzt werden oder Wasserstoff sein, der aus einem geeigneten Stoff unter den Bedingungen des erfindungsgemäßen Verfahren abgespalten wird. Als hierzu geeigneter Stoff sei beispielsweise Tetralin, Dekalin, Cyclohexan oder Isobutan genannt. Bevorzugt wird Wasserstoff als molekularer Wasserstoff eingesetzt.

Die Reaktion des erfindungsgemäßen Verfahren kann durch die folgende Formelgleichung erläutert werden :

$$(CH_3)_3C—CH_2—OH + NH_3 \longrightarrow (CH_3)_3C—CH_2—NH_2 + H_2O$$

Das erfindungsgemäße Verfahren kann beispielsweise wie folgt ausgeführt werden :

Zur diskontinuierlichen Durchführung in der Flüssigphase wird das Neopentanol, der Hydrierungskatalysator und verflüssigtes Ammoniak in einen Rührautoklaven eingefüllt. Anschließend wird Wasserstoff bis zu dem gewünschten Druck aufgepreßt. Das Gemisch wird sodann unter Rühren für einige Stunden auf die gewählte Reaktionstemperatur erhitzt. Nach dem Abkühlen und dem Entspannen wird das Reaktionsgemisch vom Katalysator, beispielsweise durch Filtration, getrennt und das hierbei entstehende Filtrat durch geeignete Methoden, wie Destillation, Extraktion oder Kristallisation, auf reines Neopentylamin aufgearbeitet.

Bei der Durchführung des erfindungsgemäßen Verfahrens in kontinuierlicher Form in der Flüssigphase wird eine Mischung aus Neopentanol und flüssigem Ammoniak, gegebenenfalls unter gleichzeitiger Einspeisung katalytischer Mengen Wasserstoff bei dem gewählten Druck und der gewählten Reaktionstemperatur in einem Druckrohr über den Hydrierungskatalysator gepumpt. Das Reaktionsgemisch kann nach Verlassen des Druckrohres in der gleichen Weise wie oben beschrieben aufgearbeitet werden. In an sich bekannter Weise kann ebenso ein Gemisch aus Neopentanol und verflüssigtem Ammoniak, gegebenenfalls unter Einspeisung katalytischer Mengen Wasserstoff im Kreislauf durch das Druckrohr über den Hydrierungskatalysator gefahren werden, wobei hinter dem Hydrierungskatalysator nur ein Teil des Kreislaufstromes entnommen und aufgearbeitet wird, während vor dem Hydrierungskatalysator ständig entsprechende Mengen Neopentanol und verflüssigtes Ammoniak, sowie gegebenenfalls katalytische Mengen Wasserstoff dem Kreislaufstrom zugefügt werden. Die Einhaltung eines bestimmten Druckes ist für die erfolgreiche Druchführung des erfindungsgemäßen Verfahrens in der bevorzugten Variante der Flüssigphasenreaktion nicht kritisch, solange gewährleistet ist, daß der Gesamtdruck des Systems deutlich über dem Dampfdruck des Reaktionsmisches bei der gewählten Reaktionstemperatur liegt.

Bei allen aufgezeigten Varianten des erfindungsgemäßen Verfahrens können das nicht verbrauchte Ammoniak und das nicht umgesetzte Neopentanol bei der Aufarbeitung des Reaktionsgemisches zurückgewonnen werden und erneut in das erfindungsgemäßen Verfahren zurückgeführt werden.

Das im erfindungsgemäßen Verfahren herstellbare Neopentylamin ist praktisch frei von höher alkylierten Aminen und braucht daher in vielen Fällen keiner zusätzlichen Trennungsoperation unterzogen zu werden. Als praktisch frei von höher alkylierten Aminen sei ein Neopentylamin mit einem Gehalt an höher alkylierten Aminen von höchstens 3 Gew.-%, bevorzugt höchstens 1 Gew.-%, bezogen auf die Menge Neopentylamin, genannt.

Es ist überraschend, daß im erfindungsgemäßen Verfahren bei relativ kleinem Ammoniak-Überschuß über das Neopentanol mit hoher Selektivität das Mono-neopentylamin erhalten wird.

Das nach dem erfindungsgemäßen Verfahren herstellbare Neopentylamin ist ein wichtiges Zwischenprodukt für die Herstellung von als Herbizide wirksamen 1-Neopentyl-tetrahydro-1,3,5-triazin-2,6-dionen nach der DE-OS 22 54 200, wobei man beispielsweise Neopentylamin mit einem 1-Alkyl-tetrahydro-1,3,5-triazin-2,6-dion umsetzt oder Neopentylamin durch Phosgenierung zunächst in das N-Neopentyl-bis-(chlorcarbonyl)-amin umwandelt und dieses mit einem substituierten Formamidin bzw. dessen Hydrochlorid umsetzt.

## Beispiel 1

Durch ein Reaktionsrohr von 50 mm Durchmesser und 340 mm Länge, das mit 1,3 kg gepilltem Raney-Nickel gefüllt ist, werden stündlich 1,5 $l_{25\,°C}$ flüssiges Ammoniak (53 Mol), 1,5 $l_{65\,°C}$ Neopentanol (13,4 Mol) mit 6,7 $l_{25\,°C}$ gasförmigem Wasserstoff (0,3 Mol) bei 200 bar gepumpt. Die Temperatur im Druckrohr beträgt 230 °C. Die Reaktionsmischung wird auf 16 bar entspannt und Ammoniak in einer Druckkolonne abdestilliert. Im Sumpf der Kolonne werden stündlich 1.183 kg eines Gemisches gewonnen, das zu 5 % aus Wasser, 22,9 % aus Neopentylamin und zu 70,3 % aus Neopentanol besteht. Die Ausbeute an Neopentylamin beträgt 24 %, die Selektivität 87,7 % der Theorie.

Beispiele 2 bis 5

werden analog Beispiel 1 durchgeführt. Die Zahlenwerte sind in der Tabelle 1 zusammengefaßt. Der Katalysator zeigt bei einem 14-tägigen Betrieb keinen Aktivitätsverlust.

Beispiel 6

Durch ein Reaktionsrohr von 50 mm Durchmesser und 340 mm Länge, das mit 0,56 kg tablettiertem Nickel auf Kieselgur (Nickelgehalt 52 %) werden stündlich 0,75 $l_{25\,°C}$ flüssiges Ammoniak (26,5 Mol), 1,5 $l_{65\,°C}$ Neopentanol (13 Mol) mit 6,7 $l_{25\,°C}$ gasförmigem Wasserstoff (0,3 Mol) bei 250 bar gepumpt. Die Temperatur im Druckrohr beträgt 250 °C. Nach Abdestillation des Ammoniaks gewinnt man im Sumpf der Kolonne stündlich 1.210 kg Gemisch, das zu 15 % aus Wasser, 71,3 % aus Neopentylamin und zu 12,8 % aus Neopentanol besteht. Die Ausbeute an Neopentylamin beträgt 76,3 %, die Selektivität 88,2 % der Theorie.

(Siehe die Tabelle 1, Seite 6)

Tabelle 1

| Beispiel | $NH_3$ | | Neopentanol | | $H_2$ | Reaktions-temperatur | Reaktions gemisch | Gehalt | | Ausbeute | Selektivität |
|---|---|---|---|---|---|---|---|---|---|---|---|
| | $l_{25\,°C}h^{-1}$ | Mol $h^{-1}$ | $l_{65\,°C}h^{-1}$ | Mol $h^{-1}$ | $l_{25\,°C}h^{-1}$ | °C | kg $h^{-1}$ | Neopentylamin % | Neopentanol % | Neopentylamin % | % der Theorie |
| 2. | 0,55 | 19,4 | 0,94 | 8,1 | 6,7 | 230 | 0,794 | 44,7 | 41,1 | 50,3 | 92,7 |
| 3 | 0,75 | 26,5 | 0,75 | 6,5 | 6,7 | 230 | 0,640 | 46,8 | 39,9 | 53,0 | 95,7 |
| 4 | 0,38 | 13,4 | 0,38 | 3,3 | 6,7 | 230 | 0,330 | 56,5 | 26,5 | 64,9 | 92,9 |
| 5 | 1,5 | 53 | 1,5 | 13 | 6,7 | 240 | 1,195 | 38,4 | 55,2 | 40,6 | 95,8 |

Beispiel 7

Ein 0,7 l fassender Rührautoklav wird mit 115 g Neopentylalkohol (Wassergehalt 9 Gew.-%) und 45 g Raney-Nickel-Eisen (Eisengehalt 15 Gew.-%) beschickt und mit Stickstoff und Wasserstoff luftfrei gespült. Dann werden bei Raumtemperatur 180 ml flüssiges Ammoniak zugefügt und Wasserstoff bis zu einem Druck von 30 bar aufgepreßt. Anschließend wird das Gemisch unter Rühren auf 265 °C erhitzt und 6 Stunden bei dieser Temperatur umgesetzt, wobei sich ein Druck von etwa 300 bar einstellt. Nach Abkühlen auf Raumtemperatur wird der Autoklav entspannt und sorgfältig entleert. Das durch Filtration vom Katalysator getrennte Reaktionsgemisch enthält außer 86 g Neopentylamin (Ausbeute 83 % der Theorie) noch 10,5 g Ausgangsmaterial (Umsatz 90 %) und 8 g Nebenprodukte, hauptsächlich Pivalinsäureamid. Das entspricht einer Selektivität von 92 %. Durch fraktionierte Destillation wird aus dem Gemisch reines Neopentylamin isoliert.

Beispiele 8 bis 10

Verwendet man bei der in Beispiel 7 beschriebenen Arbeitsweise anstelle des Raney-Nickel-Eisen-Kontaktes die in der Tabelle 2 aufgeführten Katalysatoren, so erhält man die in derselben Tabelle angegebenen Ergebnisse.

Tabelle 2

| Beispiel | Katalysator | Umsatz % | Ausbeute % d. Th. | Selektivität % |
|---|---|---|---|---|
| 8 | Raney-Nickel | 82 | 72 | 88 |
| 9 | Raney-Kobalt | 55 | 49 | 89 |
| 10 | Nickelchromit (53 % Ni, 18 % $Cr_2O_3$) | 53 | 46 | 86,5 |

**Ansprüche**

1. Verfahren zur Herstellung von Neopentylamin, das praktisch frei von höher alkylierten Aminen ist, durch Aminierung mit Ammoniak an Hydrier-/Dehydrierkatalysatoren bei erhöhter Temperatur, gegebenenfalls erhöhtem Druck und gegebenenfalls in Gegenwart von Wasserstoff, dadurch gekennzeichnet, daß man Neopentanol mit 0,5 bis 10 Mol Ammoniak umsetzt.

2. Verfahren nach Anspruch 1, dadurch gekennzeichnet, daß man die Umsetzung in flüssiger Phase bei einem Druck von mindestens 10 bar durchführt.

3. Verfahren nach Anspruch 1 und 2, dadurch gekennzeichnet, daß man die Umsetzung kontinuierlich durchführt.

4. Verfahren nach Anspruch 1 bis 3, dadurch gekennzeichnet, daß man 1 bis 5 Mol Ammoniak pro Mol Neopentanol einsetzt.

**Claims**

1. Process for the preparation of neopentylamine which is which is virtually free from amines with a higher degree of alkylation, by amination with ammonia at elevated temperature using hydrogenation/dehydrogenation catalysts, if appropriate under increased pressure and if appropriate in the presence of hydrogen, characterised in that neopentanol is reacted with 0.5 to 10 mols of ammonia.

2. Process according to Claim 1, characterised in that the reaction is carried out in the liquid phase under a pressure of at least 10 bars.

3. Process according to Claim 1 and 2, characterised in that the process is carried out continuously.

4. Process according to Claim 1 to 3, characterised in that 1 to 5 mols of ammonia are used per mol of neopentanol.

**Revendications**

1. Procédé de fabrication de néopentylamine, qui est pratiquement exempte d'amines plus fortement alcoylées, par amination avec de l'ammoniac sur des catalyseurs d'hydrogénation/déshydrogénation à température élevée, éventuellement sous pression élevée et éventuellement en présence d'hydrogène, caractérisé en ce qu'on fait réagir du néopentanol avec 0,5 à 10 moles d'ammoniac.

2. Procédé selon la revendication 1, caractérisé en ce qu'on exécute la réaction en phase liquide sous une pression d'au moins 10 bars.

3. Procédé selon les revendications 1 et 2, caractérisé en ce qu'on exécute la réaction en continu.

4. Procédé selon les revendications 1 à 3, caractérisé en ce qu'on utilise 1 à 5 moles d'ammoniac par mole de néopentanol.